# NEW EUROPEAN PATENT SPECIFICATION

(11) **EP 0 814 789 B2**
(45) Date of publication and mention of the opposition decision: **09.01.2008**
(45) Mention of the grant of the patent: 28.05.2003
(21) Application number: 96904929.5
(22) Date of filing: 01.03.1996
(51) Int. Cl.: A61K 31/135, A61K 9/00, A61P 25/16

(54) **PHARMACEUTICAL COMPOSITIONS COMPRISING MONOAMINE OXIDASE B INHIBITORS**
ARZNEIMITTEL ENTHALTEND MONOAMINOOXIDASE-B-HEMMER
COMPOSITIONS PHARMACEUTIQUES COMPORTANT DES INHIBITEURS DE MONOAMINE-OXYDASE B

(30) Priority: 02.03.1995 GB 9504235; 18.08.1995 GB 9517063
(43) Date of publication of application: 07.01.1998
(73) Proprietor: R.P. Scherer Technologies, Inc., Paradise Valley, Nevada 89119 (US)
(72) Inventor: BREWER, Francesca, Mary, Berkshire SL3 7BD (GB); JOHNSON, Edward Stewart, Ruscombe Berkshire RG10 9XG (GB); CLARKE, Anthony, Oxfordshire RG9 5AF (GB)
(74) Representative: Pearce, Anthony Richmond
(86) International application number: PCT/GB1996/000484
(87) International publication number: WO 1996/026720

(56) References cited:
- EP-A- 0 252 290
- EP-A- 0 436 492
- EP-A- 0 651 997
- WO-A-88/04552
- WO-A-90/01928
- WO-A-93/25197
- WO-A-95/11016
- WO-A-97/17067
- GB-A- 1 548 022
- US-A- 5 192 550
- US-A- 5 466 464
- Heinonen et al. (1994) Clinical Pharmacology & Therapeutics, vol. 56, no. 5, part 2, pp. 742-749
- Harris et al. (1992) Journal of Pharmaceutical Sciences, vol. 81, no. 1, pp. 1-10

## Description

This invention relates to a pharmaceutical composition, a process for preparing such a composition and the use of such a composition for the treatment of Parkinson's disease, the treatment of depression and the treatment and/or prophylaxis of Alzheimer's disease.

Selegiline ((-)-N, α - dimethyl-N-2-propynylphenethylamine) is known to be useful in the treatment of Parkinson's disease. The mechanism of action of selegiline has not been fully elucidated. However, selegiline is a potent irreversible inhibitor of monoamine oxidase, with a greater affinity for the type B form of the enzyme. Monoamine oxidase is known to play an important role in the breakdown of biological amines such as dopamine, noradrenaline and 5-hydroxytryptamine (serotonin) in the brain. It is thought that the inhibition of monoamine oxidase type B (MAO-B) may lead to enhancement of the effects of dopamine and phenylethylamine within the brain of patients with Parkinson's disease, thus leading to improved control of movement (see Gaál and Hermez, Chapter 4 in "Inhibitors of Monoamine Oxidase B, Pharmacology and Clinical Use in Neurodegenerative Disorders", edited by I. Szelenyi, (1993), Birkhäuser Verlag Basel, Switzerland, hereinafter referred to as Szelenyi.)

Selegiline is currently administered orally in the form of a conventional tablet designed to be swallowed whole or a measured amount of a conventional syrup designated to be swallowed rapidly. Accordingly, selegiline administered in this way is absorbed from the gastrointestinal tract, that is, the stomach, the small intestine and the proximal large intestine (colon), into the hepatic portal system and is presented to the liver before reaching the systemic circulation. The liver is known to be the principal site for conversion of active selegiline into metabolites, some of which are unwanted. Consequently, this first pass of absorbed selegiline through the liver results in extensive metabolism of the drug and a significant proportion of the absorbed dose of intact selegiline never reaches the systemic circulation and hence to the brain. This phenomenon is known as the "first pass effect" and results in a decrease in the bioavailability of selegiline administered in this way (see Heinonen et al, Clinical Pharmacology & Therapeutics, Vol. 56, No. 6, (1994), pp. 742-749).

Furthermore, it is known that selegiline is metabolised to produce N-desmethylselegiline, methamphetamine and amphetamine according to the following metabolic pathway:- 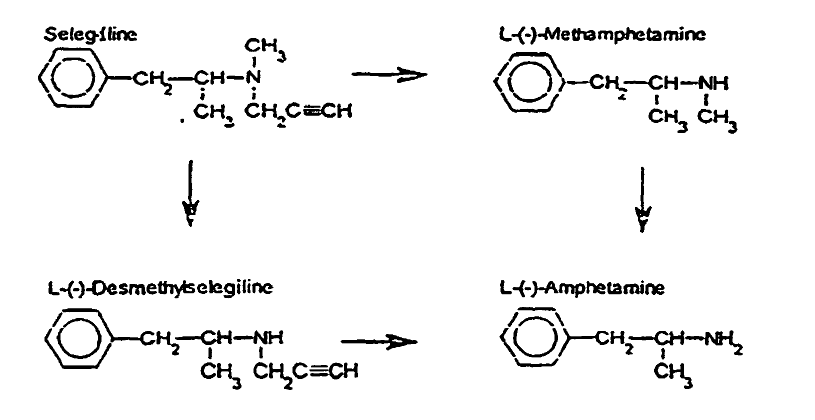 Although it has been suggested that N-desmethylselegiline may contribute to the desired inhibition of monoamine oxidases (see Heinonen et al (1993) in Chapter 10 of Szelenyi), methamphetamine and amphetamine exhibit no useful effect in Parkinson's disease. Indeed, since methamphetamine and amphetamine are both stimulants of the central nervous system and of the heart, their presence produces unwanted side-effects such as inability to sleep and cardiac arrhythmias. To minimise the central nervous system stimulant effect, currently available dosage forms of selegiline must be administered by no later than mid-day so that the unwanted stimulating effect will have subsided before the patient wishes to go to sleep at the end of the day. Clearly, this situation is far from satisfactory.

Para-fluoroselegiline is an analogue of selegiline which is also a monoamine oxidase B inhibitor and exhibits very similar pharmacological activity to that of selegiline.

Many other compounds, which are often not chemically related to selegiline, also have monoamine oxidase B-inhibiting properties, and a number of these have also been demonstrated to have utility for the treatment of Parkinson's disease, the treatment of depression and/or the treatment and/or prophylaxis of Alzheimer's disease. Among such MAO-B inhibitors may be mentioned: lazabemide [N-(2-aminoethyl)-5-chloropyridine-2-carboxamide hydrochloride]; rasagiline [2,3-dihydro-N-2-propynyl-1H-inden-1-amine]; 2-BUMP [N-(2-butyl)-N-methylpropargylamine; M-2-PP [N-methyl-N-(2-pentyl)-propargylamine] ; MDL-72145 [beta-(fluoromethylene)-3,4-dimethoxy-benzeneethanamine]; and mofegiline [(E)-4-fluoro-β-(fluoromethylene) benzene butanamine hydrochloride].

It would be highly desirable from a clinical point of view to find a way of administering such MAO-B inhibitors so that the bioavailability of the active ingredient would be enhanced, and hence monoamine oxidase B inhibition would be of more rapid onset and prolonged duration.

According to the present invention there is therefore provided a pharmaceutical composition for oral administration comprising a carrier and, as an active ingredient, a monoamine oxidase B inhibitor characterised in that the composition is in a solid fast-dispersing dosage form designed rapidly to release the active ingredient in the oral cavity so as to promote pre-gastric absorption of the active ingredient.

The term "pre-gastric absorption" is used to refer to absorption of the active ingredient from that part of the alimentary canal prior to the stomach and includes buccal, sublingual, oropharyngeal and oesophageal absorption.

The potential for the pre-gastric absorption of compositions containing MAO-B inhibitors can be assessed using the method described for selegiline in Example 3 below. This test is similar to the "buccal absorption test" which is said by Harris and Robinson in a review article (J..Pharm. Sci., 1992, vol 81, p 1-10) to be a well recognised method for evaluating buccal absorption of drugs. Thus, the test formulation containing the clinically effective dose of the MAO-B inhibitor is retained in the mouth for 1 minute before it is expectorated. The mouth is then rinsed with 3 aliquots of 25 ml of water which are similarly expectorated. The total amount of MAO-B inhibitor is then determined in the expectorated mouth washings, using a suitable analytical technique such as HPLC, and the recovered quantity of MAO-B inhibitor is subtracted from the total amount of drug initially placed in the mouth to determine the total amount of drug which has been absorbed pre-gastrically. For significant buccal absorption to have occurred it is generally preferred that at least 5% of the MAO-B inhibitor has been absorbed in 1 minute in this test, more preferably that at least 10% has been absorbed in 1 minute and most preferably at least 15% of the MAO-B inhibitor has been absorbed in 1 minute.

It is envisaged that such pre-gastric absorption will occur primarily across the mucous membranes in the mouth, pharynx and oesophagus. Accordingly, it is preferred that the composition of the invention is formulated to promote absorption of the active ingredient through the buccal, sublingual, pharyngeal and/or oesophageal mucous membranes.

It is therefore preferred that the composition of the invention should be in a form which sustains the active ingredient in contact with the buccal, sublingual, pharyngeal and/or oesophageal mucous membranes.

Clinical studies have shown that up to 82% of patients with Parkinson's disease have swallowing difficulties and many such patients tend to dribble. Accordingly, of the dosage forms listed above, fast-dispersing dosage forms are used since they will disintegrate rapidly in the mouth thereby minimising the above problems. It is therefore anticipated that such fast-dispersing dosage forms will be easier for patients to take and easier for carers to administer.

One example of a fast-dispersing dosage form is described in U.S. Patent No. 4855326 in which a melt spinnable carrier agent, such as sugar, is combined with an active ingredient and the resulting mixture spun into a "candy-floss" preparation. The spun "candy-floss" product is then compressed into a rapidly dispersing, highly porous solid dosage form.

U.S. Patent No. 5120549 discloses a fast-dispersing matrix system which is prepared by first solidifying a matrix-forming system dispersed in a first solvent and subsequently contacting the solidified matrix with a second solvent that is substantially miscible with the first solvent at a temperature lower than the solidification point of the first solvent, the matrix-forming elements and active ingredient being substantially insoluble in the second solvent, whereby the first solvent is substantially removed resulting in a fast-dispersing matrix.

U.S. Patent No. 5079018 discloses a fast-dispersing dosage form which comprises a porous skeletal structure of a water soluble, hydratable gel or foam forming material that has been hydrated with water, rigidified in the hydrated state with a rigidifying agent and dehydrated with a liquid organic solvent at a temperature of about 0°C or below to leave spaces in place of hydration liquid.

Published International Application No. WO 93/12769 (PCT/JP93/01631) describes fast-dispersing dosage forms of very low density formed by gelling, with agar, aqueous systems containing the matrix-forming elements and active ingredient, and then removing water by forced air or vacuum drying.

U.S. Patent No. 5298261 discloses fast-dispersing dosage forms which comprise a partially collapsed matrix network that has been vacuum-dried above the collapse temperature of the matrix. However, the matrix is preferably at least partially dried below the equilibrium freezing point of the matrix.

Published International Application No. WO 91/04757 (PCT/US90/05206) discloses fast-dispersing dosage forms which contain an effervescent disintegration agent designed to effervesce on contact with saliva to provide rapid disintegration of the dosage form and dispersion of the active ingredient in the oral cavity.

The term "fast-dispersing dosage form" therefore encompasses all the types of dosage form described in the preceding paragraphs. However, it is particularly preferred that the fast-dispersing dosage form is of the type described in U.K. Patent No. 1548022, that is, a solid fast-dispersing dosage form comprising a network of the active ingredient and a water-soluble or water-dispersible carrier which is inert towards the active ingredient, the network having been obtained by subliming solvent from a composition in the solid state, that composition comprising the active ingredient and a solution of the carrier in a solvent.

It is preferred that the composition of the invention disintegrates within 1 to 10 seconds, particulary 2 to 8 seconds, of being placed in the oral cavity.

In the case of the preferred type of fast-dispersing dosage form described above, the composition will preferably contain, in addition to the active ingredient, matrix forming agents and secondary components. Matrix forming agents suitable for use in the present invention include materials derived from animal or vegetable proteins, such as the gelatins, dextrins and soy, wheat and psyllium seed proteins; gums such as acacia, guar, agar, and xanthan; polysaccharides; alginates; carboxymethylcelluloses; carrageenans; dextrans; pectins; synthetic polymers such as polyvinylpyrrolidone; and polypeptide/protein or polysaccharide complexes such as gelatin-acacia complexes.

Other matrix forming agents suitable for use in the present invention include sugars such as mannitol, dextrose, lactose, galactose and trehalose; cyclic sugars such as cyclodextrin; inorganic salts such as sodium phosphate, sodium chloride and aluminium silicates; and amino acids having from 2 to 12 carbon atoms such as a glycine, L-alanine, L-aspartic acid, L-glutamic acid, L-hydroxyproline, L-isoleucine, L-leucine and L-phenylalanine.

One or more matrix forming agents may be incorporated into the solution or suspension prior to solidification. The matrix forming agent may be present in addition to a surfactant or to the exclusion of a surfactant. In addition to forming the matrix, the matrix forming agent may aid in maintaining the dispersion of any active ingredient within the solution or suspension. This is especially helpful in the case of active agents that are not sufficiently soluble in water and must, therefore, be suspended rather than dissolved.

Secondary components such as preservatives, antioxidants, surfactants, viscosity enhancers, colouring agents, flavouring agents, pH modifiers, sweeteners or taste-masking agents may also be incorporated into the composition. Suitable colouring agents include red, black and yellow iron oxides and FD & C dyes such as FD & C blue No. 2 and FD & C red No. 40 available from Ellis & Everard. Suitable flavouring agents include mint, raspberry, liquorice, orange, lemon, grapefruit, caramel, vanilla, cherry and grape flavours and combinations of these. Suitable pH modifiers include citric acid, tartaric acid, phosphoric acid, hydrochloric acid and maleic acid. Suitable sweeteners include aspartame, acesulfame K and thaumatin. Suitable taste-masking agents include sodium bicarbonate, ion-exchange resins, cyclodextrin inclusion compounds, adsorbates or microencapsulated actives.

Preferred compositions in accordance with this invention include as the active MAO-B inhibitor a compound of the general formula: 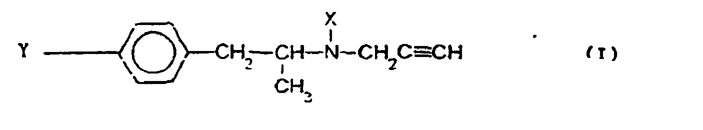 or an acid addition salt thereof, in which X represents a hydrogen atom or, preferably, a methyl group and Y represents a fluorine or, preferably, a hydrogen atom. It is particularly preferred that X is methyl and Y is hydrogen i.e. that the active MAO-B inhibitor is selegiline.

Selegiline or para-fluoroselegiline which is absorbed by pre-gastric absorption from a composition in accordance with this invention passes straight into the systemic circulatory system thereby avoiding first pass metabolism in the liver. Accordingly, the initial rapid production of unwanted metabolites is reduced and the bioavailability of active selegiline or para-fluoroselegiline is increased. This results in a number of advantages. For instance, the increased bioavailability of active selegiline or para-fluoroselegiline means that the dose of selegiline or para-fluoroselegiline may be reduced whilst still producing the desired beneficial effect. This will result in a further decrease in the production of unwanted metabolites and, in the case of selegiline, a corresponding reduction in the stimulatory effect of methamphetamine and amphetamine on the central nervous system and heart. Consequently, no restrictions on dose timing are required for the compositions of the invention.

In the case of selegiline and its analogues of formula I above, the active ingredient preferably is present in the composition in an amount of from 1 to 30%, more preferably 1 to 20%, by weight of the composition. It is also preferred that the active ingredient is present in the composition in an amount of from 0.25 to 30 mg, more preferably 0.5 to 10 mg and, especially, 1 to 5 mg.

In the case of other MAO-B inhibitors these also will be present in concentrations which are clinically effective.

According to another aspect of the invention there is provided a process for preparing a pharmaceutical composition as defined above which comprises bringing a carrier into association with the MAO-B inhibiting active ingredient.

The invention also provides, in a further aspect, a composition as defined above for use in the treatment of Parkinson's disease.

As mentioned above, selegiline and para-fluoroselegiline are both inhibitors of monoamine oxidase B. The preferred substrate for monoamine oxidase B is phenylethylamine, a chemical which occurs naturally in the brain. Phenylethylamine is structurally very closely related to amphetamine and recent studies have indicated that phenylethylamine may act as a neuromodulator promoting elevation of mood. Indeed, this is borne out by the fact that patients suffering from depression have been found to have sub-normal levels of phenylethylamine in the brain.

In view of this, it has been suggested that monoamine oxidase B inhibitors, such as selegiline, may be useful in the treatment of depression since inhibition of monoamine oxidase B will result in increased levels of phenylethylamine. However, in practice, it has generally been found that high doses, typically 30-60mg per day for long periods (e.g. 6 weeks), of selegiline are required to elevate the mood of depressed patients. Such high doses are associated with non-specific inhibition of both monoamine oxidase A and monoamine oxidase B, selective inhibition of monoamine oxidase B being a feature of low doses (10mg or less) of selegiline. Although monoamine oxidase A has very little effect on the metabolism of phenylethylamine, it has been suggested that inhibition of monoamine oxidase A may produce an anti-depressant effect by inhibiting deamination of norepinephrine and 5-hydroxytryptamine (serotonin), deficits of which are also associated with depression. However, inhibition of monoamine oxidase A can produce undesirable cardiovascular effects and tyramine-induced hypertensive crisis (the so-called "cheese effect"). Accordingly, the use of such high doses of selegiline or other MAO-B inhibitors to combat depression is clearly far from ideal.

As an alternative, it has been proposed to administer a lower dose of selegiline (10mg) in conjunction with phenylalanine (250mg), which is the dietary precursor of phenylethylamine. In this combination, selegiline inhibits the production of monoamine oxidase B thereby inhibiting the deamination of phenylethylamine and phenylalanine stimulates phenylethylamine synthesis. This results in increased levels of phenylethylamine in the brain and therefore concomitant elevation of mood. However, two agents need to be given and the onset of the anti-depressant effect is still slow.

To date, no studies have shown consistent anti-depressant activity using low doses of selegiline alone. However, it has now been found that, if selegiline or, by implication, other MAO-B inhibiting compound is formulated in a composition according to the present invention, an increase in the amount of phenylethylamine occurs in the body and thereby a good anti-depressant effect may be achieved at dose levels associated with selective inhibition of monoamine oxidase B. Moreover, an earlier onset of effect is likely to be achieved than with existing formulations and, in the case of selegiline, the low dose levels result in lower levels of unwanted metabolites and therefore a reduction in their associated side effects.

According to another aspect of the invention there is therefore provided the use of a composition as defined above for the manufacture of a medicament for the treatment and/or prophylaxis of depression.

Recent studies have also shown that selegiline and other MAO-B inhibitors have a positive effect in the treatment and/or prophylaxis of Alzheimer's disease since this condition is also associated with a marked increase in levels of monoamine oxidase B in the brain when compared with age-matched controls. Accordingly, since formulation of selegiline and, by implication, other MAO-B inhibitors in a composition according to the present invention has been shown to increase bioavailability of the active ingredient, such compositions may be especially effective in the treatment and/or prophylaxis of Alzheimer's disease whilst minimising unwanted metabolites and associated side effects.

According to a further aspect of the invention there is therefore provided the use of a composition as defined above for the manufacture of a medicament for the treatment and/or prophylaxis of Alzheimer's disease.

Since it is well-known that demented patients with Alzheimer's disease may not comply with their treatment regimen, may be uncooperative and even spit out tablets, the fast-dispersing dosage forms of the invention are particularly useful since, not only will they disintegrate rapidly in the mouth thereby reducing the opportunity for ejection of the complete dosage form, but it has also been established that a significant portion of the active ingredient is absorbed into the body from this dosage form even if a portion is expectorated.

The invention is further illustrated by the following examples.

### Example 1

### Preparation of a fast-dispersing dosage form of selegiline

### (a) Preparation of selegiline hydrochloride 2.0% dispersion

Gelatin (720g) and mannitol (540g) were dispersed in a portion of purified water (15.73kg)by mixing thoroughly in the bowl of a vacuum mixer. The remaining water (1.5 litres) was added under vacuum while mixing using an anchor stirrer. The mix was then heated to 40°C ± 2°C and homogenised for ten minutes. The mix was cooled down to room temperature. When cooled, a 4500g portion of the mix was removed into a stainless steel vessel and glycine (360g), aspartame (90g), grapefruit flavour (54g), Opatint yellow (54g), citric acid (90g) and selegiline hydrochloride (360g) were then added sequentially to this portion while homogenising using a bench top homogeniser. The remainder of the mix was transferred into a second stainless steel vessel. The mix was homogenised for ten minutes using a bench top mixer to dissolve the drug. Once dispersion of the colouring agent was complete, the homogenised portion of the mix in the first vessel was returned to the mixer bowl together with the mix from the second vessel. The combined mixes were then mixed for at least 20 minutes. The bulk dispersion was then homogenised to ensure that mixing was complete.

### (b) Preparation of selegiline hydrochloride 5mg units

250mg of the selegiline hydrochloride 2.0% dispersion formed in (a) above was dosed into each one of a series of pre-formed blister pockets having a pocket diameter of 12 mm. The blister laminate comprised 200µm PVC/30µm PE/PVDC 90g per square metre. The product was frozen immediately in a liquid nitrogen freeze tunnel, The frozen product was then stored below - 20°C for a minimum of 24 hours prior to freeze-drying in a freeze drier using a drying temperature of + 20°C and a chamber pressure of 0.5 mbar. The freeze-dried units were then inspected for the presence of critical defects and the remainder of the batch sealed with lidding foil consisting of a paper/foil laminate (20µm aluminium). Each blister was then coded with a batch number and overwrapped in a preformed sachet by placing the blister in the sachet and sealing the open end of the sachet completely. Each sachet was then labelled with the product name, batch number, date of manufacture and suppliers name.

Each unit dosage form had the following composition:

| Ingredient | Weight (mg) | % by wt of composition |
|---|---|---|
| Purified Water USP/EP* | 218.500 | 87.4 |
| Selegiline Hydrochloride | 5.000 | 2.0 |
| Gelatin EP/USNF | 10.000 | 4.0 |
| Mannitol BP/USP | 7.500 | 3.0 |
| Aspartame EP/USN | 1.250 | 0.5 |
| Grapefruit Flavour 502.106/A | 0.750 | 0.3 |
| Glycine USP | 5.000 | 2.0 |
| Citric Acid EP/USP | 1.250 | 0.5 |
| Opatint AD-22901 yellow | 0.750 | 0.3 |
| | 250.000 | 100.0 |

| | | |
|---|---|---|
| * Signifies removed during the lyophilisation process. | | |

### Example 2

### Comparative pharmacokinetic study

The aim of this experiment was to compare the bioavailability of the selegiline hydrochloride formulation of Example 1 with the commercially available tablet formulation of selegiline hydrochloride sold under the registered Trade Mark "Movergan" by Asta Medica AG, Weismüllerstrasse 45, 6000 Frankfurt am Main, Germany.

An open label, randomised, 2-way crossover, volunteer study was performed as follows. Twenty four subjects of either sex, aged between 45 and 71 years, giving written informed consent underwent a thorough medical examination to establish their fitness to participate in the study. Subjects received study treatment in the order dictated by a pre-determined randomisation schedule. Subjects were given either the formulation of Example 1 or the "Movergan" formulation. Blood samples for determination of pharmacokinetic parameters were taken at baseline (immediately before drug administration), then after 0.25, 0.5, 0.75, 1, 1.5, 2, 2.5, 3, 4, 5, 6, 8, 10, 12, 24, 48, 72 and 96 hours. The study procedures were repeated two weeks later, when subjects were crossed-over to receive their second drug administration. Selegiline hydrochloride was administered as single 10mg doses (made up from 2 x 5 mg tablets) of the formulation of Example 1 or of the "Movergan" formulation.

Assays were performed to determine the concentrations of selegiline, N-desmethylselegiline, methamphetamine and amphetamine in each of the blood plasma samples. The following pharmacokinetic parameters were determined for all four analysed substances: bioavailability (as measured as the area under the curve (AUC) of the drug concentrations/time plot), Cmax (the maximum plasma concentration achieved and Tmax (the time-point at which Cmax was observed).

The results are shown in graphical form in Figures 1 to 4 where each figure is a plot of the concentration of a specific compound in a blood plasma sample versus the time at which the sample was taken for the formulation of Example 1 (Example 1) and the tablet formulation sold under the registered Trade Mark "Movergan" (Movergan). In Figure 1, the specific compound is selegiline. In Figure 2, the specific compound is N-desmethylselegiline. In Figure 3, the specific compound is methamphetamine. In Figure 4, the specific compound is amphetamine.

The results are shown in numerical form in Table 1 below. In this table, the references to N-desmethylselegiline, methamphetamine and amphetamine are to the L-(-)- isomers of these compounds.

**TABLE 1**

| | Selegiline | N-desmethylselegiline | Methamphetamine | Amphetamine |
|---|---|---|---|---|
| AUC | | | | |
| Example 1 | 6.93 | 36.58 | 215.43 | 104.85 |
| Movergan | 0.83 | 35.60 | 234.91 | 108.01 |
| | | | | |
| Cmax | | | | |
| Example 1 | 5.17 | 14.47 | 8.90 | 3.01 |
| Movergan | 0.86 | 17.36 | 10.59 | 3.54 |
| | | | | |
| Tmax | | | | |
| Example 1 | 0.33 | 0.71 | 2.40 | 5.40 |
| Movergan | 0.58 | 0.72 | 2.16 | 4.16 |
| | | | | |
| Key | AUC | Area under the plasma concentration-time curve (ng.h/ml) | | |
| | Cmax | Maximum plasma concentration (ng/ml) | | |
| | Tmax | Time to maximum plasma concentration (h) | | |

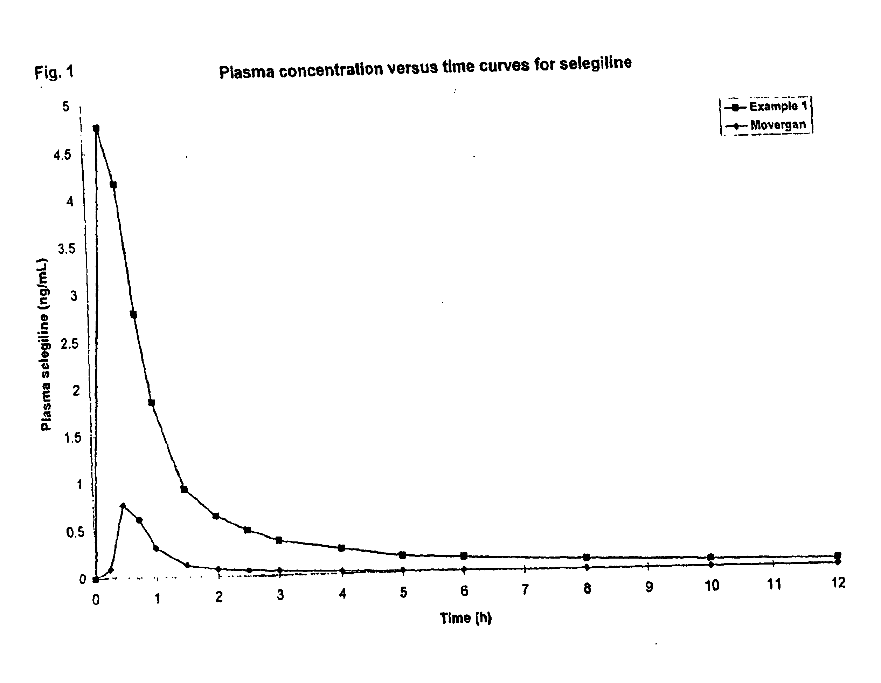

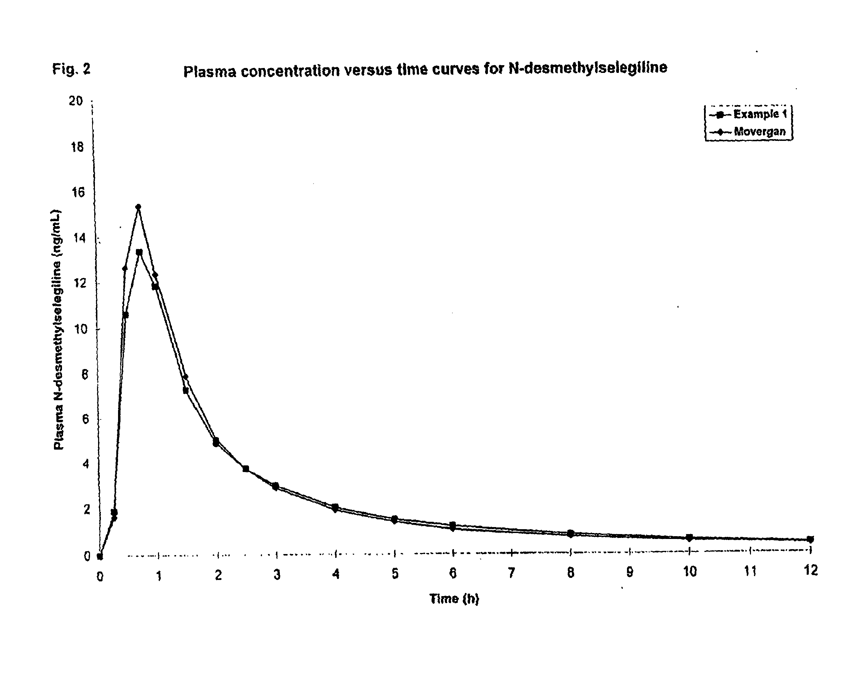

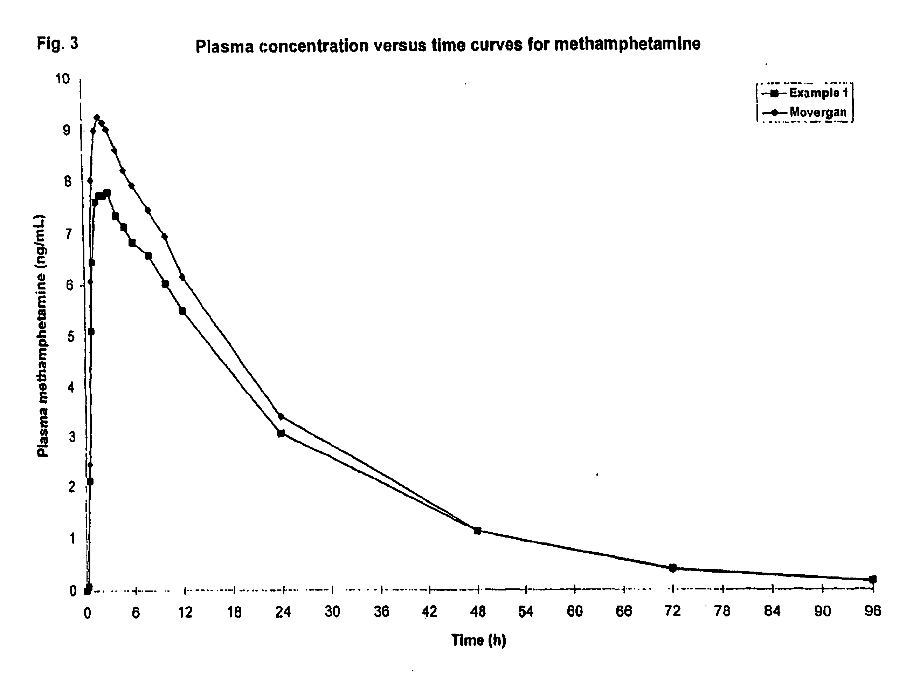

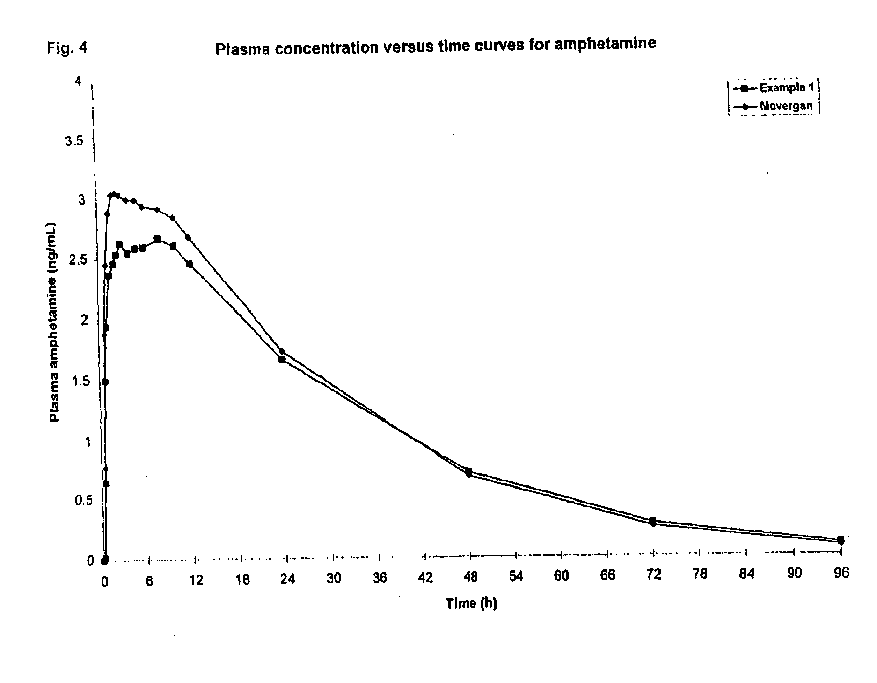

From Figure 1 to 4 and Table 1, it is apparent that the bioavailability of selegiline from the formulation of Example 1 is more than eight times that of selegiline from the "Movergan" formulation despite the fact that both formulations contained the same amount of active ingredient. Also, the bioavailability of N-desmethylselegiline is very similar for both formulations. The bioavailabilities of methamphetamine and amphetamine, which are known not to contribute to the therapeutic effect, are very similar for Example 1 and the "Movergan" formulation. However, in view of the much greater bioavailability of selegiline from the formulation of Example 1, it is envisaged that the dose of selegiline could be significantly reduced thereby significantly reducing the quantity of unwanted central nervous system and cardiac stimulant metabolites and undesired side-effects caused by them whilst still achieving the desired levels of selegiline in plasma and hence the desired therapeutic effect associated with monoamine oxidase B inhibition.

In Table 1, the ratio of the area under the plasma concentration-time curve (AUC) for selegiline and the AUC for N-desmethylselegiline was 0.0233 for the "Movergan" formulation, indicating clearly the extensive metabolism of selegiline when administered in an existing dosage form. The corresponding AUC ratio for Example 1 in Table 1 was 0.1894. This demonstrates that pre-gastric absorption of selegiline results in a greater proportion of the administered dose being absorbed in the unmetabolised form. It demonstrates further that the selegiline:N-desmethylselegiline AUC ratio can be used as another indicator of the degree of pre-gastric absorption in selegiline-containing compositions in accordance with this invention. It is generally preferred that the ratio of the selegiline AUC to the N-desmethylselegiline AUC should be greater than 0.05, more preferably greater than 0.075 and most preferably greater than 0.10.

### Example 3

### Pre-gastric Absorption Study

The aim of this study was to assess the sublingual absorption of selegiline hydrochloride formulations produced according to Example 1. The pharmacokinetic profile of selegiline hydrochloride from the commercially available US tablet formulation sold under the registered trademark "Eldepryl" by Somerset Pharmaceuticals Inc. 777 South Harbour Island Boulevard, Suite 880, Tampa, Florida 33602, served as a control for the degree of gastro-intestinal absorption of selegiline. In addition, the study was designed to compare the urinary excretion over 24 hours of phenylethylamine and 5-hydroxyindoleacetic acid (5-HIAA) from the subjects to whom such formulations had been administered.

This study was an open-label randomised 3-way crossover volunteer study and was performed as follows:

Eleven subjects of either sex aged between 45 and 62 years giving written informed consent underwent a thorough medical examination to establish their fitness to participate in the study. Subjects received each of the following treatments in the order dictated by a pre-determined randomisation schedule:-
1) 2 x 5mg Eldepryl tablets taken with 150ml water (Eldepryl (10mg))
2) 2 x 5mg selegiline tablets produced according to Example 1 kept in the mouth for 1 minute and then expectorated and the mouth rinsed with 3 x 25ml water and then expectorated (Example 1 (2.96mg))
3) 2 x 5mg selegiline tablets produced according to Example 1 kept in the mouth for 1 minute and then swallowed (Example 1 (10mg)).

Blood samples for determination of pharmacokinetic parameters were taken at baseline (immediately before drug administration) and then after 0.08, 0.16, 0.25, 0.5, 0.75, 1, 1.5, 2, 3, 4, 6 and 12 hours. Urine samples were taken immediately before drug administration and during the periods 0-2 hours, 2-4 hours, 4-6 hours, 6-12 hours and 12-24 hours.

Assays were performed to determine the concentration of selegiline in each of the blood plasma and urine samples and the concentration of phenylethylamine and 5-hydroxyindoleacetic acid (5HIAA) was measured in each of the urine samples. Selegiline was also measured in saliva and mouth washings.

Phenylethylamine is the preferred substrate for monoamine oxidase B (MAO-B) and consequently its excretion has been shown to rise when MAO-B is inhibited. 5HIAA is a breakdown product formed by the action of MAO-A on 5-hydroxytryptamine (serotonin). When MAO-A is inhibited, the 5HIAA level excreted has been shown to decline.

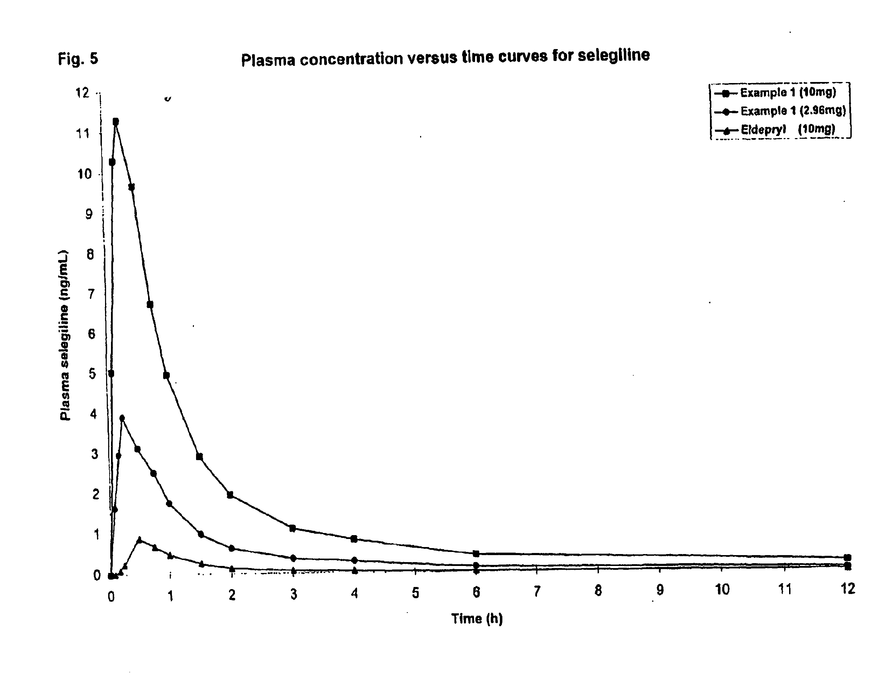

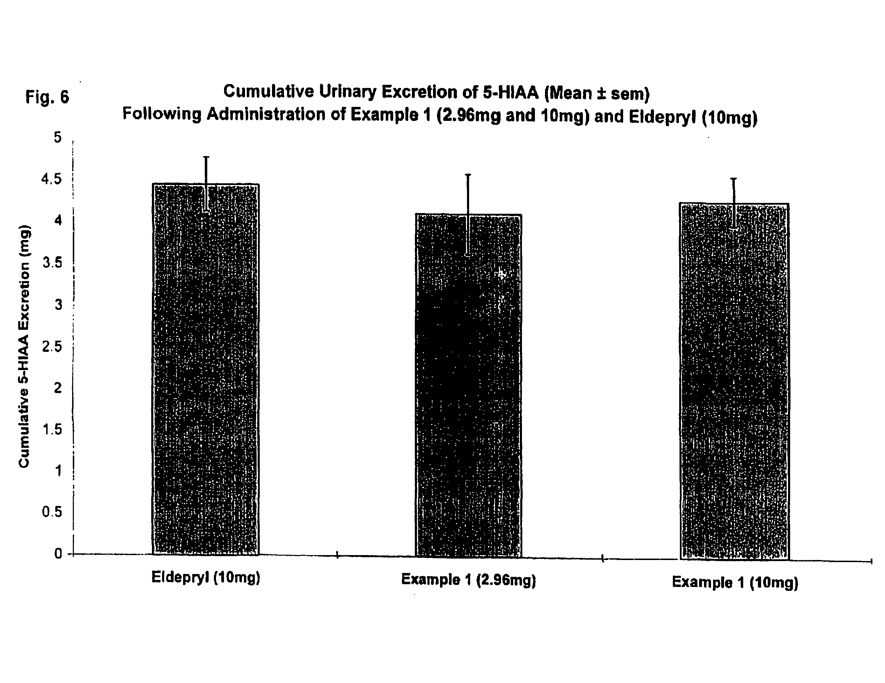

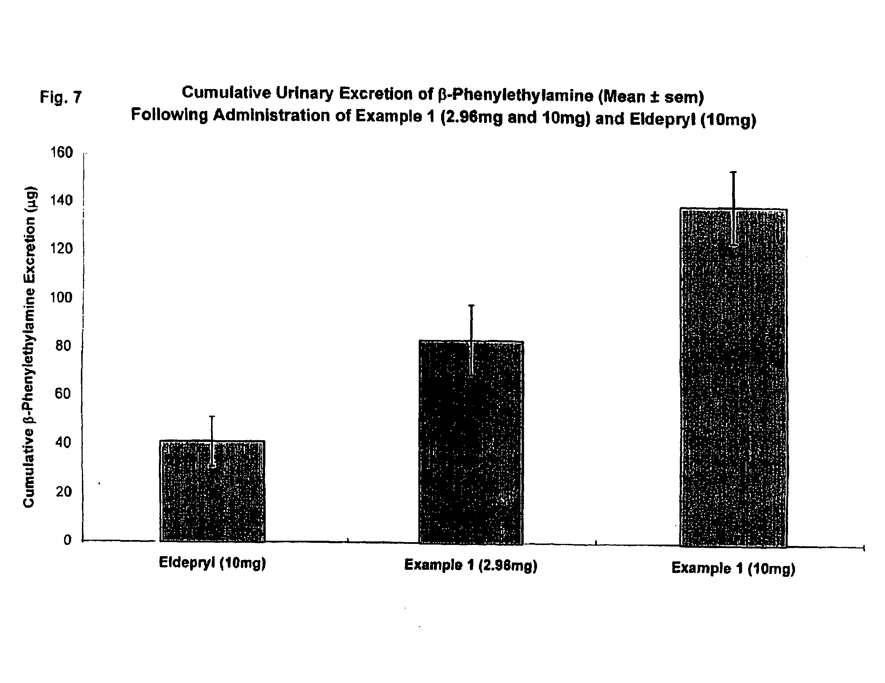

The results from the study are shown in graphical form in Figures 5, 6 and 7. When the tablets produced according to Example 1 were kept in the mouth for 1 minute and the saliva expectorated, an average concentration equivalent to 7.04mg selegiline hydrochloride was measured in the mouth washings. Thus an average of 2.96mg selegiline hydrochloride was absorbed pregastrically with this treatment. Subjects therefore received 2.96mg or 10mg of selegiline hydrochloride from the 10mg formulation produced according to claim 1 and 10mg selegiline from the Eldepryl formulation. Figure 5 is a plot of concentration of selegiline in a blood plasma sample versus the time at which the sample was taken for both expectorated and swallowed formulations produced according to Example 1 (Example 1 (equivalent to 2.96mg) and Example 1 (10mg) respectively) and the 10mg tablet formulation sold under the registered Trade Mark "Eldepryl". Figure 6 shows the cumulative 5-hydroxyindoleacetic acid excretion in urine over 24 hours. Figure 7 shows the cumulative phenylethylamine excretion in urine over 24 hours.

From Figure 5, it is apparent that the bioavailability of selegiline from both the 2.96mg (expectorated) equivalent and 10mg (swallowed) doses produced according to Example 1 is much greater than that of selegiline from the "Eldepryl" formulation despite the fact that one formulation (Example 1 (10mg "swallowed")) contained the same amount of active ingredient as the "Eldepryl" formulation and the expectorated treatment contained less than one third of the amount of active ingredient as the "Eldepryl" formulation. Moreover, it is apparent from Figure 7 that this enhanced bioavailability is associated with a dose-related increase in the urinary excretion of phenylethylamine. This was an unexpected result as increased phenylethylamine excretion is caused by inhibition of monoamine oxidase B and it was hitherto believed that 10mg of selegiline in standard tablet form (i.e. "Eldepryl") would be sufficient to cause maximal inhibition of monoamine oxidase B during the first 24 hours. In addition, the higher rate of excretion of phenylethylamine in Figure 7 for Example 1 (10mg "swallowed") and Example 1 (2.96mg "expectorated") than for the "Eldepryl" formulation indicates a faster rate of monoamine oxidase B inhibition than with the former compositions and consequently a possible earlier alleviation of symptoms of Parkinson's disease, Alzheimer's disease and depressed mood than for the "Eldepryl" formulation.

Lack of inhibition of monoamine oxidase A by the Example 1 (10mg "swallowed") and Example 1 (2.96mg "expectorated") treatments was confirmed by analysis of the urine samples for concentration of 5-hydroxyindoleacetic acid, which is the metabolite of 5-hydroxytryptamine (serotonin) which is a principal substrate for monoamine oxidase A (see Figure 6). Urinary concentrations of 5-hydroxyindoleacetic acid were similar for the Example 1 (10mg "swallowed"), Example 1 (2.96mg "expectorated") and the standard "Eldepryl" tablet formulations, showing that the selegiline formulations produced according to Example 1 did not cause greater MAO-A inhibition than standard tablets despite the much increased selegiline bioavailability.

Once again, in view of the greater bioavailability of selegiline from the Example 1 (10mg "swallowed") and Example 1 (2.96mg "expectorated") formulations, it is envisaged that the dose of selegiline could be significantly reduced thereby significantly reducing the quantity of undesired metabolites with their associated side effects whilst still achieving the desired therapeutic effects associated with inhibition of monoamine oxidase B.

The following examples further exemplify formulations which can be prepared using the process described in Example 1 which will promote pre-gastric absorption of selegiline and other MAO-B inhibitors:

### Example 4

| Ingredient | Weight (mg) | % by wt of composition |
|---|---|---|
| Purified Water EP/USP* | 221.625 | 88.65 |
| Selegiline Hydrochloride | 5.000 | 2.00 |
| Gelatin EP/USNF | 11.250 | 4.50 |
| Mannitol EP/USP | 8.125 | 3.25 |
| Aspartame EP/USNF | 1.250 | 0.50 |
| Grapefruit Flavour 502.106/A | 0.750 | 0.30 |
| Citric Acid EP/USP | 1.250 | 0.50 |
| Opatint AD-22901 Yellow | 0.750 | 0.30 |
| Total | 250.000 | 100.00 |

| | | |
|---|---|---|
| *Signifies removed during the lyophilisation process | | |

### Example 5

| Ingredient | Weight (mg) | % by wt of composition |
|---|---|---|
| Purified Water EP/USP* | 224.125 | 89.65 |
| Selegiline Hydrochloride | 5.000 | 2.00 |
| Gelatin EP/USNF | 9.375 | 3.75 |
| Mannitol EP/USP | 7.500 | 3.00 |
| Grapefruit Flavour 502.106/A | 0.750 | 0.30 |
| Citric Acid EP/USP | 1.250 | 0.50 |
| Opatint AD-22901 Yellow | 0.750 | 0.30 |
| Acesulfame K | 1.250 | 0.50 |
| Total | 250.000 | 100.00 |

| | | |
|---|---|---|
| *Signifies removed during the lyophilisation process | | |

### Example 6

| Ingredient | Weight (mg) | % by wt of composition |
|---|---|---|
| Purified Water EP/USP* | 219.500 | 87.80 |
| Selegiline Hydrochloride | 5.000 | 2.00 |
| Gelatin EP/USNF | 10.000 | 4.00 |
| Mannitol EP/USP | 7.500 | 3.00 |
| Aspartame EP/USNF | 1.000 | 0.40 |
| Glycine USP | 2.500 | 1.00 |
| Citric Acid EP/USP | 1.250 | 0.50 |
| Opatint AD-22901 Yellow | 0.750 | 0.30 |
| Lemon Lime 59.15/AP | 2.500 | 1.00 |
| Total | 250.000 | 100.00 |

| | | |
|---|---|---|
| *Signifies removed during the lyophilisation process | | |

### Example 7

| Ingredient | Weight (mg) | % by wt of composition |
|---|---|---|
| Purified Water EP/USP* | 223.625 | 89.45 |
| Selegiline Hydrochloride | 5.000 | 2.00 |
| Gelatin EP/USNF | 10.000 | 4.00 |
| Mannitol EP/USP | 7.500 | 3.00 |
| Aspartame EP/USNF | 0.750 | 0.30 |
| Grapefruit Flavour 502.106/A | 0.750 | 0.30 |
| Citric Acid EP/USP | 1.250 | 0.50 |
| Opatint AD-22901 Yellow | 0.750 | 0.30 |
| Sodium Methyl Parabens EP/USNF | 0.250 | 0.10 |
| Sodium Propyl Parabens EP/USNF | 0.125 | 0.05 |
| Total | 250.000 | 100.00 |

| | | |
|---|---|---|
| *Signifies removed during the lyophilisation process | | |

### Example 8

| Ingredient | Weight (mg) | % by wt of composition |
|---|---|---|
| Purified Water EP/USP* | 219.125 | 87.65 |
| Selegiline Hydrochloride | 5.000 | 2.00 |
| Gelatin EP/USNF | 10.625 | 4.25 |
| Mannitol EP/USP | 6.875 | 2.75 |
| Aspartame EP/USNF | 1.250 | 0.50 |
| Glycine USP | 5.000 | 2.00 |
| Grapefruit Flavour 502.106/A | 0.750 | 0.30 |
| Citric Acid EP/USP | 0.625 | 0.25 |
| Opatint AD-22901 Yellow | 0.750 | 0.30 |
| Total | 250.000 | 100.00 |

| | | |
|---|---|---|
| *Signifies removed during the lyophilisation process | | |

### Example 9

| Ingredient | Weight (mg) | % by wt of composition |
|---|---|---|
| Purified Water EP/USP* | 216.750 | 86.7 |
| Selegiline Hydrochloride | 5.000 | 2.0 |
| Gelatin EP/USNF | 10.000 | 4.0 |
| Mannitol EP/USP | 7.500 | 3.0 |
| Aspartame EP/USNF | 1.250 | 0.5 |
| Glycine USP | 3.750 | 1.5 |
| Citric Acid EP/USP | 1.250 | 0.5 |
| Opatint AD-22901 Yellow | 0.750 | 0.3 |
| Acesulfame K | 1.250 | 0.5 |
| Lemon Lime 59.15/AP | 2.500 | 1.0 |
| Total | 250.000 | 100.00 |

| | | |
|---|---|---|
| *Signifies removed during the lyophilisation process | | |

### Example 10

| Ingredient | Weight (mg) | % by wt of composition |
|---|---|---|
| Purified Water EP/USP* | 215.875 | 86.35 |
| Mofegiline | 12.000 | 4.80 |
| Gelatin EP/USNF | 10.000 | 4.00 |
| Mannitol EP/USP | 8.125 | 3.25 |
| Aspartame EP/USNF | 1.250 | 0.50 |
| Grapefuit Flavour 502.106/A | 0.750 | 0.30 |
| Glycine USP | 1.250 | 0.50 |
| Opatint AD-22901 Yellow | 0.750 | 0.30 |
| Total | 250.000 | 100.00 |

| | | |
|---|---|---|
| *Signifies removed during the lyophilisation process | | |

### Example 11

| Ingredient | Weight (mg) | % by wt of composition |
|---|---|---|
| Purified Water EP/USP* | 797.500 | 79.75 |
| Lazabemide | 100.000 | 10.00 |
| Gelatin EP/USNF | 45.000 | 4.50 |
| Mannitol EP/USP | 35.000 | 3.50 |
| Lemon Lime 59.15/AP | 5.000 | 0.50 |
| Glycine USP | 10.000 | 1.00 |
| Aspartame EP/USNF | 7.500 | 0.75 |
| Total | 1000.000 | 100.00 |

| | | |
|---|---|---|
| *Signifies removed during the lyophilisation process | | |

## Claims

1. A pharmaceutical composition for oral administration comprising a carrier and, as an active ingredient, a monoamine oxidase B inhibitor of the general formula 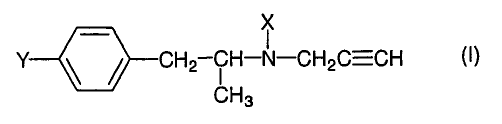 in which X represents a hydrogen atom or a methyl group and Y represents a fluorine or hydrogen atom, **characterised in that** the composition is in a solid fast-dispersing dosage form designed rapidly to release the active ingredient in the oral cavity so as to promote pre-gastric absorption of said monoamine oxidase B inhibitor.

2. A composition according to claim 1, in which X represents a methyl group and Y represents a hydrogen atom.

3. A composition according to claim 1 or claim 2, in which the active ingredient is present in an amount of from 1 to 30 % by weight of the composition.

4. A composition according to any one of claims 1 to 3, in which the active ingredient is present in an amount of from 0.25 to 30 mg.

5. A composition according to any preceding claim in which the solid fast-dispersing dosage form comprises a network of the active ingredient and a water-soluble or water-dispersible carrier which is inert towards the active ingredient, the network having been obtained by subliming solvent from a composition in the solid state, that composition comprising the active ingredient and a solution of the carrier in a solvent.

6. A composition according to any preceding claim, in which the composition disintegrates within 1 to 10 seconds of being placed in the oral cavity.

7. A pharmaceutical composition for oral administration comprising a carrier, and selegiline as an active ingredient, **characterised in that** the composition is in the form of a solid fast-dispersing dosage form comprising a network of selegiline and a water-soluble or water-dispersible carrier which is inert towards selegiline, the network having been obtained by subliming solvent from a composition in the solid state, that composition comprising selegiline and a solution of the carrier in a solvent.

8. A pharmaceutical composition for oral administration comprising selegiline in a solid fast-dispersing dosage form which disintegrates within 1 to 10 seconds of being placed in the oral cavity.

9. A composition as defined in any one of the preceding claims for use in the treatment of Parkinson's disease.

10. Use of a composition as defined in any one of claims 1 to 8 for the manufacture of a medicament for the treatment and/or prophylaxis of depression.

11. Use of a composition as defined in any one of claims 1 to 8 for the manufacture of a medicament for the treatment and/or prophylaxis of Alzheimer's disease.

12. A process for preparing a pharmaceutical composition according to any one of the claims 1 to 8 which comprises bringing a carrier into association with said active ingredient.

## Patentansprüche

1. Eine pharmazeutische Zusammensetzung zur oralen Anwendung umfassend einen Träger und, als einen aktiven Inhaltsstoff, einen Monoaminooxidase-B-Hemmer, mit der folgenden Formel, 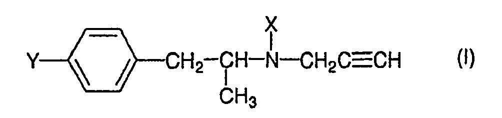 **dadurch gekennzeichnet, dass** die Zusammensetzung in einer festen, schnell dispergierenden Dosierungsform vorliegt, so angelegt, um den aktiven Inhaltsstoff schnell in der Mundhöhle freizusetzen, zur Förderung einer Absorption des Monoaminooxidase-B-Hemmers vor dem Magen.

2. Eine Zusammensetzung nach Anspruch 1, bei welcher X für eine Methylgruppe steht und Y für ein Wasserstoffatom steht.

3. Eine Zusammensetzung nach Anspruch 1 oder Anspruch 2, in welcher der aktive Inhaltsstoff in einer Menge von 1 bis 30 Gewichtsprozent der Zusammensetzung vorhanden ist.

4. Eine Zusammensetzung nach einem der Ansprüche 1-3, in welcher der aktive Inhaltsstoff in einer Menge von 0,25 bis 30 mg vorhanden ist.

5. Eine Zusammensetzung nach einem der vorstehenden Ansprüche, in welcher die feste, schnell dispergierende Dosierungsform ein Netzwerk umfasst aus dem aktiven Inhaltsstoff und einem wasserlöslichen oder in Wasser dispergierbaren Träger, welcher inert ist gegenüber dem aktiven Inhaltsstoff, wobei das Netzwerk erhalten wurde durch Sublimieren eines Lösungsmittels von einer Zusammensetzung im festen Zustand, wobei diese Zusammensetzung den aktiven Inhaltsstoff und eine Lösung des Trägers in einem Lösungsmittel umfasst.

6. Eine Zusammensetzung nach einem der vorstehenden Ansprüche, wobei die Zusammensetzung innerhalb von 1 bis 10 Sekunden nach Platzierung in der Mundhöhle zerfällt.

7. Eine pharmazeutische Zusammensetzung zur oralen Anwendung umfassend einen Träger und Selegilin als einen aktiven Inhaltsstoff, **dadurch gekennzeichnet, dass** die Zusammensetzung in der Form einer festen, schnell dispergierenden Dosierungsform vorliegt, umfassend ein Netzwerk aus Selegilin und einem wasserlöslichen oder in Wasser dispergierbaren Träger, welcher inert ist gegenüber Selegilin, wobei das Netzwerk erhalten wurde durch Sublimieren eines Lösungsmittels von einer Zusammensetzung im festen Zustand, wobei diese Zusammensetzung Selegilin und eine Lösung des Trägers in einem Lösungsmittel umfasst.

8. Eine pharmazeutische Zusammensetzung zur oralen Anwendung, welche Selegilin in einer festen, schnell dispergierenden Dosierungsform umfasst, welche innerhalb von 1 bis 10 Sekunden nach Platzierung in der Mundhöhle zerfällt.

9. Eine Zusammensetzung, wie in einem der vorstehenden Ansprüche definiert, zur Verwendung bei der Behandlung der Parkinson-Krankheit.

10. Verwendung einer Zusammensetzung, wie in einem der Ansprüche 1 bis 8 definiert, zur Herstellung eines Medikamentes zur Behandlung und/oder Prophylaxe von Depressionen.

11. Verwendung einer Zusammensetzung, wie in einem der Ansprüche 1 bis 8 definiert, zur Herstellung eines Medikamentes zur Behandlung und/oder Prophylaxe der Alzheimer-Krankheit.

12. Ein Verfahren zur Präparation einer pharmazeutischen Zusammensetzung nach einem der Ansprüche 1 bis 8, welches die Assoziierung eines Trägers mit dem aktiven Inhaltsstoff umfasst.

## Revendications

1. Composition pharmaceutique pour administration orale, comprenant un véhicule et, en tant qu'ingrédient actif, un inhibiteur de la monoamine oxydase B, avec la formule suivante, 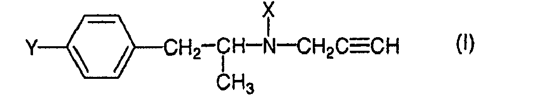 **caractérisée en ce que** la composition est sous une forme posologique solide à dispersion rapide élaborée pour libérer rapidement l'ingrédient actif dans la cavité buccale de façon à activer l'absorption prégastrique dudit inhibiteur de monoamine oxydase B.

2. Composition selon la revendication 1, dans laquelle X représente un groupe méthyle et Y représente un atome d'hydrogène.

3. Composition selon la revendication 1 ou la revendication 2, dans laquelle l'ingrédient actif est présent en une quantité de 1 à 30% en poids de la composition.

4. Composition selon l'une quelconque des revendications 1 à 3, dans laquelle l'ingrédient actif est présent en une quantité de 0,25 à 30mg.

5. Composition selon l'une quelconque des revendications précédentes, dans laquelle la forme posologique solide à dispersion rapide comprend un réseau de l'ingrédient actif et d'un véhicule hydrosoluble ou dispersible dans l'eau qui est inerte vis-à-vis de l'ingrédient actif, le réseau ayant été obtenu en sublimant le solvant à partir d'une composition à l'état solide, cette composition comprenant l'ingrédient actif et une solution du véhicule dans un solvant.

6. Composition selon l'une quelconque des revendications précédentes, la composition se désintégrant en l'espace de 1 à 10 secondes après avoir été placée dans la cavité buccale.

7. Composition pharmaceutique pour administration orale comprenant un véhicule, et de la sélégiline en tant qu'ingrédient actif, **caractérisée en ce que** la composition est sous la forme d'une forme posologique solide à dispersion rapide comprenant un réseau de sélégiline et d'un véhicule hydrosoluble ou dispersible dans l'eau qui est inerte vis-à-vis de la sélégiline, le réseau ayant été obtenu en sublimant le solvant à partir d'une composition à l'état solide, cette composition comprenant de la sélégiline et une solution du véhicule dans un solvant.

8. Composition pharmaceutique pour administration orale comprenant de la sélégiline sous une forme posologique solide à dispersion rapide qui se désintègre en l'espace de 1 à 10 secondes après avoir été placée dans la cavité buccale.

9. Composition telle que définie dans l'une quelconque des revendications précédentes pour l'utilisation dans le traitement de la maladie de Parkinson.

10. Utilisation d'une composition telle que définie dans l'une quelconque des revendications 1 à 8 pour la fabrication d'un médicament pour le traitement et/ou la prophylaxie de la dépression.

11. Utilisation d'une composition telle que définie dans l'une quelconque des revendications 1 à 8 pour la fabrication d'un médicament pour le traitement et/ou la prophylaxie de la maladie d'Alzheimer.

12. Procédé pour préparer une composition pharmaceutique selon l'une quelconque des revendications 1 à 8, qui comprend la mise en association d'un véhicule avec ledit ingrédient actif.
